# EUROPEAN PATENT SPECIFICATION

(11) **EP 1 644 558 B1**
(45) Date of publication and mention of the grant of the patent: **03.04.2019**
(21) Application number: 04760840.1
(22) Date of filing: 27.04.2004
(51) Int. Cl.: C25D 5/00, C25D 21/18, C23C 16/00, C25B 3/00, C02F 1/72, C25B 1/02, H01F 3/06, H01M 6/00, C07C 303/44, C25D 5/18, H01M 10/08, H01M 10/26, C25F 1/02, C25D 3/02, C25F 1/04

(54) **HIGH PURITY ELECTROLYTIC SULFONIC ACID SOLUTIONS**
HOCHREINE ELEKTROLYTISCHE SULFONSÄURELÖSUNGEN
SOLUTIONS D'ACIDE SULFONIQUE ELECTROLYTIQUES DE HAUTE PURETE

(30) Priority: 12.05.2003 US 469764 P
(43) Date of publication of application: 12.04.2006
(73) Proprietor: Arkema Inc., King Of Prussia, PA 19406 (US)
(72) Inventor: MARTYAK, Nicholas, Michael, Doylestown, PA 18901 (US); NOSOWITZ, Martin, Paoli, PA 19301 (US); SMITH, Gary, S., Collegeville, PA 19426 (US); JANNEY, Patrick, Kendall, Ridley Park, PA 19078 (US); OLLIVIER, Jean-Marie, F-64260 Buzy (FR)
(74) Representative: Schaefer, Anne-Sophie
(86) International application number: PCT/US2004/012887
(87) International publication number: WO 2004/101860

(56) References cited:
- EP-A- 0 505 692
- US-A- 4 895 977
- US-A1- 2001 053 449
- US-B1- 6 187 169
- CLARKE R L ET AL: "INTRODUCING CERIUM BASED HIGH ENERGY REDOX BATTERIES" EESAT CONFERENCE PAPERS, 15 April 2002 (2002-04-15), pages 1-6, XP002906984

## Description

### BACKGROUND OF THE INVENTION

### FIELD OF THE INVENTION

The present invention relates to high purity sulfonic acids and their use in electrochemical processes, methods for preparing such high purity acids, methods for preparing high purity metal-sulfonate or sulfonic acid solutions, and products formed by using such methods and solutions.

### PRIOR ART

Electrochemical processes may contain acid electrolytes to impart conductivity and thus lower the required voltage, aid in the dissolution of metal (e.g., formation of metal salts) and metal oxides (e.g., descaling), aid in the deposition of metals from aqueous solutions (e.g., electrodeposition), and may be used in the synthesis of conductive polymers to provide proton conduction and in batteries to aid in the dissolution and solvation of metals such as zinc and lead.

There are many acids that may be used in the above applications such as sulfuric, nitric, phosphoric, sulfamic, and hydrochloric acid as well as phosphonic and sulfonic acids. The choice of the acids is very dependent upon the application and the purity of the acid. For example, hydrochloric acid is not typically used to descale ferrous-based metals due to its propensity for pitting corrosion in the metal. Sulfuric acid is not often used in the electrodeposition of silver or lead-based alloys due to the likelihood of metal precipitation in this acid.

There are also usually several grades or purities of acids and the choice of grade is often again dependent upon the industrial application. For example, sulfuric acid may be purchased as technical grade, reagent grade, electronic grade and microelectronic grade. Technical grade has the most impurities and is therefore not used in metal electrodeposition solutions as required in the microelectronic field.

Recently, sulfonic acids, including alkane sulfonic acids, have gained acceptance in many commercial applications due in large part to (a) their ability to solubilize metals that are insoluble in acids such as sulfuric acid, are typically non-corroding acids like hydrochloric, (b) being reducing acids unlike nitric acid and (c) being more stable than sulfamic acid at low to moderate pH and elevated temperatures.

Additionally, improvements in electroplating solutions and techniques have been made (a) to meet increased standards for plating and (b) to be able to plate under more highly difficult circumstances.

Most improvements focused on the use of organic additives such as suppressor additives, accelerator additives and leveling additives and the like to obtain the desired results.

However, even with the improvements in electroplating processes, circumstances may exist that can lead to plating defects due to inadequate coverage in recess areas such as in the vias or trenches or through-holes in electronic devices, poor corrosion resistance of the deposited metal, too high a residual stress leading to cracking in the metal coating or a rough, commercially unacceptable deposit. These defects can occur as a result of an imbalance of the organic additives intended to obtain the desired metallic coating.

Also, the useful lifetime of many metal electrodeposition electrolytes is dependent upon the breakdown of the organic additives, particularly the sulfur-containing accelerator additive.

It is well known in the industry that by having typical low valent sulfur-containing accelerator type additive such as those used in acid copper, nickel, cobalt and iron solutions, uniform plating of particularly low to high aspect ratio vias and microvias and other difficult-to-plate electronic features such as through-holes in printed circuit boards is possible.

Typical accelerator or brightener additives contain one or more low valent sulfur atoms, and typically without any nitrogen atoms and a molecular weight of about 1500 or less. In all cases, the low-valent sulfur accelerator or brightener decomposes to impart the desired effects.

However, combinations of various low-valent sulfur compounds is typically unwanted due to competitive interactions at the surface of the work piece.

Therefore, it would be desirable to control the concentration of the wanted low-valent sulfur additive in narrow ranges, often in the milligram per liter range, and to avoid the interaction with unwanted low-valent sulfur impurity molecules from the acid make-up solution.

The synthesis of conductive polymers such as polyaniline may also employ sulfonic acids to impart conductivity through protonic doping. Choi and co-workers in Synthetic Metals showed aniline polymerization in the presence of dodecylbenzesulfonic acid exhibited good electrical conductivity. Dominis and co-workers in Synthetic Metals, studied the synthesis of polyaniline in a solution containing nonylnaphthalene sulfonic acid and found that the sulfonic acid aids in the solvation of the conductive polymer in organic media. There was no mention of the purity of the sulfonic acid used in these studies.

During the electrodeposition of nickel, iron and cobalt, additives may be employed in the aqueous electroplating solution to impart brightness to the metal deposit, decrease the stress in the metal coating, increase the corrosion protection of the underlying substrate or to achieve a desired esthetic appearance.

The composition of the additives used in the electroplating solution is dependent upon the desired metal coating. However, in general, additives for these metal electroplating baths may contain sulfur moieties as described by Lowenheim in Modern Electroplating, 3rd Ed. Class 1 brighteners reduce the grain size of the metal coating but also incorporate a small, about 0.03%, amount of sulfur. In the corrosion protection of steel, a duplex nickel coating is required whereby the underlying nickel (e.g., close to the steel) must not contain any sulfur in the deposit. The nickel electrolyte is formulated such that the additives do not contain reducible sulfur compounds that may eventually be co-deposited in the nickel. The top nickel layer of the duplex contains small amounts of sulfur and corrodes preferentially compared to the underlying sulfur-free nickel coating. Bodnevas and Zahavi in Plating and Surface Finishing, (Dec. 1994, pg. 75) showed the effects of sulfur-bearing additives on the internal stress of nickel deposit, the incorporation of sulfur into the nickel coating and the relationship between the sulfur-additive concentration in the solution and the incorporation of elemental sulfur in the nickel deposit plated from sulfate-based solutions. If the nickel electrolytes are made using impure sulfonic acids such as those containing reduced or easily reducible sulfur compounds, the likelihood of sulfur incorporation increases significantly thus affecting the resultant stress in the deposit, the brightness of the metallic coating and the corrosion properties.

In depositing low stress nickel coatings, for use in aerospace applications, from sulfamate-based solutions, no sulfur is co-deposited in the coating even if using a conventional sulfur-bearing stress reducing agent such as 1,3,5, naphthalenetrisulfonic acid (NTS). In NTS, sulfur has an oxidation state of +6 and is not easily reduced. However, reducible sulfur compounds, if present in impure sulfonic acids, may alter the grain size of the nickel deposit and consequentially alter the stress in the metallic nickel deposit.

The synthesis of sulfonic acids may be complex and several undesirable impurities may be present in the desired sulfonic acids leading to difficulties in using sulfonic acids in electrochemical processes. Sulfonic acids may be made via the oxidation of the corresponding thiol, by hydrolysis of alkanesulfonyl halide, or by the oxidation of dimethyldisulfide. Various impurities may also be made during the oxidation or hydrolysis reaction and thus must be removed prior to use. Many low valent sulfur compounds such as sulfur (II) or sulfur (IV) or higher valent sulfur molecules such as sulfur (VI) compounds that are susceptible to reduction and are present in the sulfonic acid may produce a stench or odor, interfere with the ongoing electrochemical process or alter the final product.

Low valent or easily reducible sulfur compounds in an acidic medium may also produce an undesired sulfur stench. This stench comes from the formation of minute amounts of hydrogen sulfide, dimethylsulfide or sulfur dioxide. These materials are unwanted and dangerous during electrochemical processes.

It thus would be desirable to have new electrochemical compositions based on high purity sulfonic acids. It would be particularly desirable to have new sulfonic acid compositions that can be effectively used with metals of strong reducing capabilities such as tin, zinc and iron without deleterious effects such as odor and defects in the metal or polymer deposit. Such compositions could be used in electrodeposition, batteries, conductive polymers and de-scaling applications.

### SUMMARY OF THE INVENTION

The invention is defined in current claim 1. Preferred embodiments are defined in claims 2 to 21.

Compositions of the present invention are useful in processes that are tailored to meet more stringent requirements in electrodeposition of electronic circuits, conductive polymers, batteries and descaling work.

The compositions of the invention are useful for depositing a metal, modifying or cleaning a scaled metal surface or for use as acid electrolytes in batteries or during the synthesis of conductive polymers.

### DETAILED DISCRIPTION OF THE INVENTION

Compositions of the invention suitably contain a sulfonic acid with a low concentration of reduced or easily reducible sulfur species that have the ability to produce an odor during the dissolution of a metal or interfere with the electrolysis process.

It has now been found that compositions of high purity sulfonic acids, which when employed in electrochemical processes, produce no odor during make-up and use and commercially acceptable metal deposits, cleaned metal surfaces and polymer coatings are obtained.

This invention focuses on the unexpected superiority of using high purity sulfonic acids in electrochemical processes such as in electrodeposition, batteries, conductive polymers and descaling.

The sulfonic acids used in this invention have low concentrations of reduced sulfur compounds and sulfur compounds in high oxidation states that are susceptible to reduction, that are capable of producing an undesirable odor during use, during make-up of metal-sulfonate salts. These undesirable low valent sulfur compounds are also capable of producing rough or commercially unacceptable electrodeposits produced from the metal-sulfonate salt solutions. If present in sufficiently high enough concentration, the low valent sulfur compound may be incorporated into the metallic coating and thus alter the corrosion protection of the metals. The low valent sulfur compounds may also interfere with the polymerization of organic monomers to form conductive polymers.

Sulfonic acid electrolytes of the invention are characterized in significant part by comprising a low concentration of reduced sulfur compounds and sulfur compounds in a higher oxidation state that are susceptible to reduction by an active metal or during electrolysis to low valent sulfur compounds (odor-causing impurities) such as sulfides.

In particular, high purity solutions of the present invention have a total concentration of reduced sulfur compounds less than 5 mg/liter, still more preferably at least less than 1 mg/liter.

In addition, other compounds may be used with the high purity sulfonic acid solutions of the present invention, such as metal salts of the sulfonic acid or other inorganic or organic metal salts, plating additives such as grain refiners, levelers, accelerators, conductivity salts, buffers, chelating agents.

The sulfonic acids of this invention may also contain oxidizing agents, reducing agents, sequestering agents, surfactants, emulsifying agents, viscosity modifiers, wetting agents, lubricants, soaps and a co-solvent. The choice of additional additives or buffers is dependent upon the operation of choice such as electrodeposition, descaling, organic monomer polymerization, energy storage devices and mixture of the above compounds may be used to effective the desired result.

The application also relates to the production of metal salts using the high purity sulfonic acid. Metal sulfonates solutions may be produced from a variety of processes such as the electro-dissolution of a metal into the high purity sulfonic acid, dissolution of various metal oxides, carbonates or other metal salts.

The application also includes articles of manufacture employing sulfonic acids of this invention, including electronic packaging devices such as printed circuit boards, multichip modules, semiconductor integrated circuits, mechanical-electronic machine devices (i.e., MEMS devices) and the like, batteries such as zinc-halogen, zinc-lanthanide, and vanadium-based energy storage devices, conductive polymers such as polyaniline and polypyrrole, zinc galvanized steel, tin-plated steel, automobile parts, aerospace parts and other articles of manufacturing using the sulfonic acids described in this invention.

The high purity sulfonic acids of the present invention may be prepared whereby the impure sulfonic acid is first treated to remove the low-valent sulfur molecules by adding to the impure sulfonic acid an oxidizing agent to increase the valency of the impurity or low valent sulfur molecules, mixing the sulfonic acid with the oxidizing at temperatures between 25°C to about 95°C for a sufficient time to complete or nearly complete the oxidation of the low valent sulfur compounds to the higher-state sulfur compounds. Optionally, one may heat the sulfonic acid to elevated temperatures to remove or destroy any residual oxidizing agent, and optionally, adding activated carbon powder to remove residual impurities remaining in the sulfonic acid.

Many different oxidizing agents may be used such as hydrogen peroxide, nitric acid, permanganate ion, an anodic electric current, monoperoxysulfate, an aqueous solution of chlorine, or a halogen in a solution of a carboxylic acid.

Sulfonic acids used in this invention contain impurities such as low valent sulfur (II) or sulfur (IV) compounds or higher valent sulfur (VI) compounds that are susceptible to reduction and may form odorous sulfur compounds. For example, dimethylsulfone, CH₃SO₂CH₃, maybe reduced to sulfur dioxide, SO₂, or dimethylsulfide (DMS), CH₃SCH₃. The oxidation state of sulfur in DMSO₂ is six whereas in SO₂, sulfur is in the +four oxidation state and in DMS it is two. MMTS, CH₃SO₂SCH₃, which has both a sulfur(II) and a sulfur(VI) entity may be reduced by metals or electrochemically to dimethyldisulfide, (CH₃SSCH₃), whereby both sulfurs are now in the +two oxidation state.

The sulfonic acid used in this invention are characterized in large part as an alkyl monosulfonic acid, an alkyl polysulfonic acid or an aryl mono or polysulfonic acid with low concentrations of reduced or easily reducible sulfur compounds and are introduced as: 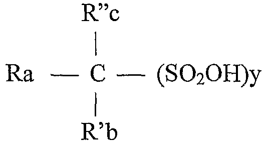 wherein a+b+c+y equals 4; R, R' and R" are the same or different and each independently may be hydrogen, phenyl, Cl, F, Br, I, CF₃ or a lower alkyl group such as (CH₂)n where n is from 1 to 23 and that is unsubstituted or substituted by oxygen, Cl, F, Br, I, CF₃, -SO₂OH.

The preferred alkyl sulfonic acids are methanesulfonic, ethanesulfonic and propanesulfonic acids; the preferred alkyl polysulfonic acids are methanedisulfonic acid, monochloromethanedisulfonic acid, dichloromethanedisulfonic acid, 1,1-ethanedisulfonic acid, 2-chloro-1,1-ethanedisulfonic acid, 1,2-dichloro-1,1-ethanedisulfonic acid, 1,1-propanedisulfonic acid, 3-chloro-1,1-propanedisulfonic acid, 1,2-ethylene disulfonic acid, 1,3-propylene disulfonic acid, trifluormethanesulfonic acid, butanesulfonic acid, perfluorobutanesulfonic acid, and pentanesulfonic acid; and the preferred aryl sulfonic acids are phenylsulfonic, phenolsulfonic, para-toulenesulfonic, and xylenesulfonic acids; or mixtures thereof.

The sulfonic acid may be used from a concentration range from less than 1 g/l to 1480 g/l, more preferably, at a concentration of about 10 to about 700 grams per liter of solution, still more preferably, at a concentration of from about 30 to about 500 grams per liter of solution.

The sulfonic acid solution of the invention may have a pH that is between 2 to 13.

The sulfonic acid of the invention may be an aqueous solution wherein the acid solution is a mixture of a sulfonic acid with other inorganic or organic acids.

As discussed above, electroplating solutions, batteries and de-scaling formulations of the application are particularly effective in solubilizing active metals such as tin, nickel, cobalt, iron, zinc and more noble metals such as copper and silver without the production of an unwanted odor.

Pure sulfonic acid or metal sulfonate solutions of the invention generally comprise at least one soluble metal salt, an acid electrolyte, optionally a buffering agent, optionally plating bath additives generally referred to as leveling agents, brighteners, suppressors, conductivity salts and the like, and optionally a halogen ion. More particularly, electroplating compositions of the invention preferably contain a metal salt of an alkyl or aryl sulfonic acid of this invention; an electrolyte, preferably an acidic aqueous solution such as a sulfonic acid solution with optionally a buffering agent, optionally plating bath additives generally referred to as leveling agents, brighteners, suppressors and the like, and optionally a halogen ion and the like.

A variety of metal salts may also be in the sulfonic acid electrolyte of this invention, the exact composition is dependent upon the desired final metal finish and properties of the metallic coating. Metal sulfonate salts selected from Group 1B, 2B, 3A, 3B, 4A, 4B, 5A, 5B, 6B, 7B, 8B, lanthanide and actinide metals of the periodic table and ammonium ion or mixtures thereof may be used.

Metal alkanesulfonate salts may be employed in the subject electroplating solutions wherein the alkanesulfonic acid of the anionic portion of the metal salt and any free acid are introduced as an alkyl or aryl sulfonic acid having the formula as above. The preferred alkyl sulfonic acids, alkyl polysulfonic acids and aryl sulfonic acids are as above. Metal methanesulfonates are particularly preferred metal salts.

The term metal sulfonate in this invention includes metals and metal alloys. Metals and alloys may be selected from Group 1B, 2B, 3A, 3B, 4A, 4B, 5A, 5B, 6B, 7B, and 8B, lanthanide and actinide metals of the periodic table as well as ammonium ion or mixtures thereof.

The metal salt of an alkyl or aryl sulfonic acid usually has the formula: 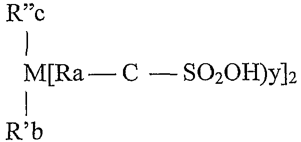
wherein a+b+c+y equals 4,
R, R' and R" are the same or different and each independently may be hydrogen, phenyl, Cl, F, Br, I, CF₃ or a C₁₋₉ alkyl group such as (CH₂)n where n is from 1 to 23 and that is unsubstituted or substituted by oxygen, Cl, F, Br, I, CF₃, or -SO₂OH.

A metal sulfonate salt may be suitably present in a relatively wide concentration range in the aqueous sulfonic acid of the invention. Preferably, a metal salt will be employed at a concentration from about less than 1 grams per liter to about 500 grams per liter of plating solution, more preferably at a concentration of from about 20 to about 300 grams per liter of plating solution, still more preferably at a concentration of from about 40 to about 175 grams per liter of plating solution.

In addition, metal salts of sulfate, nitrate, sulfamate, and chloride from Group 1B, 2B, 3B, 4A, 4B, 5A, 5B, 6B, 7B, 8B, lanthanide and actinide metals of the periodic table and ammonium ion or mixtures thereof may also be used in this invention.

Preferred metal methanesulfonates are those of zinc, copper, nickel, ferrous, alkali earth metals and alkaline earth metals.

The electrolyte may also contain free acid to increase solution conductivity such as that needed in efficient batteries and electrodeposition processes. The preferred free acid has the same anion as the metal salt anion but mixtures of acids are also within the scope of this invention. Acids such as sulfuric, hydrochloric, sulfamic, acetic, propioinic, phosphonic, tartartic, oxalic, phosphonic and nitric among others may be used in this invention.

The free acid concentration ranges from about less than 1 gram per liter to about 900 gram per liter, more preferably at a concentration of from about 2 to about 700 grams per liter of plating solution, still more preferably at a concentration of from about 10 to about 500 grams per liter of solution.

The invention also includes sulfonate solutions that are substantially or completely free of added inorganic or organic acids and may be neutral or basic (e.g. pH of at least less than about 13 to about 7). Such sulfonate electrolyte compositions are suitably prepared in the same manner with the same components as other compositions disclosed herein but without an added free sulfonic acid.

Electrochemical sulfonic solutions of the invention may employ a halide ion source, particularly a chloride ion source. Examples of other suitable halides include fluoride, bromide and iodide. A wide range of halide ion concentrations (if a halide ion is employed) may be suitably utilized, e.g. from about 0 (where no halide ion employed) to 500 g/l, in solution, more preferably from about 10 to about 400 g/l of halide ion source in the sulfonic acid solution.

In addition to the metal salts, aqueous acid electrolytes of the invention optionally may contain a variety of other components commonly known in the electrodeposition industry. These components are often referred to as additives such as suppressors agents, accelerator agents, leveling agents and the like. The use of a buffering agent in combination with a suppressor agent, an accelerator or brightener additive is particularly preferred and provides surprisingly enhanced plating performance, particularly in hard to plate features.

A buffering agent may be used with the sulfonic acids of this invention. The buffering agent regulates the pH during electrolysis. Examples of suitable buffering agents include monocarboxylic, dicarboxylic and tricarboxylic acid such as citric acid, tartaric acid, potassium sodium tartrate, amino acids, oxalic acid, nitrogen-containing carboxylic acids and phosphonic acids. Preferred is boric acid.

Useful brighteners include those of the following formula:

XO₃S-R-SH

XO₃S-R-S-S-R-SO₃X

and

XO₃-Ar-S-S-Ar-SO₃X

where in the above formulae R is an optionally substituted alkyl group, and preferably is an alkyl group having from 1 to 6 carbon atoms, more preferably is an alkyl group having from 1 to 4 carbon atoms; Ar is an optionally substituted aryl group such as optionally substituted phenyl or naphthyl; and X is a suitable counter ion such as ammonium, sodium or potassium. Some specific suitable brighteners include e.g. n,n-dimethyl-dithiocarbamic acid-(3-sulfopropyl)ester; 3-mercapto- propylsulfonic acid-(3-sulfopropyl)ester; 3-mercaptopropylsulfonic acid (sodium salt); carbonic acid-dithio-o-ethylester-s-ester with 3-mercapto-1-propane sulfonic acid (potassium salt); bissulfopropyl disulfide; 3-(benzthiazolyl- s-thio)propyl sulfonic acid (sodium salt); pyridinium propyl sulfobetaine; 1-sodium-3-mercaptopropane-1-sulfonate; sulfoalkyl sulfide compounds disclosed in U.S. Pat. No. 3,778,357; the peroxide oxidation product of a dialkyl amino-thiox-methyl-thioalkanesulfonic acid; and combinations of the above. Additional suitable brighteners are also described in U.S. Pat. Nos. 3,770,598,4,374,709,4,376,685, 4,555,315, and 4,673,469, all incorporated herein by reference. Particularly preferred brighteners for use in the plating compositions of the invention are n,n-dimethyl-dithiocarbamic acid-(3-sulfopropyl)ester and bis-sodium-sulfonopropyldisulfide.

Surfactants useful in the present invention include e.g. amines such as ethoxylated amines, polyoxyalkylene amines and alkanol amines; amides; polyglycol-type wetting agents, such as polyethylene glycols, polyalkylene glycols and polyoxyalkyene glycols; high molecular weight polyethers; polyethylene oxides (mol. wt. 100,000 to 3 million); block copolymers of polyoxy- alkyenes; alkylpolyether sulfonates; complexing surfactants such as alkoxylated diamines; Particularly suitable surfactants for plating compositions of the invention are commercially available polyethylene glycol copolymers, including polyethylene glycol copolymers.

Wetting agents may be used with the sulfonic acids of this invention. The wetting agents may be selected from cationic, anionic or non-ionic molecules.

The invention may also use complexing agents for metals particularly if the sulfonic acid of the invention is used in electrodeposition or de-scaling processes. Examples of suitable complexing agents include monocarboxylic, dicarboxylic and tricarboxylic acid such as citric acid, tartaric acid, potassium sodium tartrate, amino acids, particularly glycine, oxalic acid, alklylamines, alkylalkanol amine, EDTA and phosphonic acids.

Electrochemical processes of the invention may be carried out from less than ambient temperatures to elevated temperatures. Preferably, the electrochemical processes are used from about -20°C to above 95°C depending upon the processes in use.

The sulfonic acid solution may be stagnant or is preferably agitated during use such as by using an air sparger, physical movement of the work piece, ultrasonic radiation, impingement or other suitable methods.

Electrolysis is preferably conducted at a current density ranging from less than 1.08 x 10⁻⁴ to 3.23A/cm², depending upon the process and the electrolyte characteristics.

Electrolysis times may range from about less than one minute to greater than twenty-four hours or more, depending on the difficulty of the work piece and the desired finish.

The invention described also includes the use of direct, pulse or periodic current waveforms to effectively produce a commercially acceptable metal or polymer coating.

The invention described may also use a soluble or an insoluble or inert electrode material.

A wide variety of substrates may be electroplated, de-scaled or coated with a conductive polymer with the compositions of the invention, as discussed above. The compositions of the invention are particularly useful to electroplate difficult work pieces, such as circuit board substrates with small diameter and low aspect vias or through-holes, integrated circuits with low aspect ratio vias, integrated circuits with high aspect ration microvias and other electronic features. The plating compositions of the invention also will be particularly useful for plating integrated circuit devices, such as formed semiconductor devices and the like. The sulfonic acids of this invention will also be useful for the de-scaling of metals. The sulfonic acids of this invention will also be useful as an electrolyte in batteries and other energy storage devices and during the polymerization of organic monomers to form conductive polymers.

The foregoing description of the invention is merely illustrative thereof, and it is understood that variations and modifications can be effected without departing from the scope of the invention as set forth in the following claims.

### Example 1

A. Commercially available 70% methanesulfonic acid (MSA) that contains a high concentration of methylmethanethiosulfonate, [MMTS, (CH3SO2SCH3)] was purified by the following procedure:
   (1) 250 ml of 70% methanesulfonic acid containing 12 parts-per-million (ppm) was placed into a 500 ml beaker.
   (2) 30% hydrogen peroxide solution, 1.125 grams, was added and the MSA was heated to 60°C, 140 degrees Fahrenheit for three hours.
   (3) The methanesulfonic acid solution was cooled to room temperature and analyzed for MMTS.

The concentration of MMTS after the hydrogen peroxide treatment was non-detectable by gas chromatography methods. After purification the MSA was diluted to 15% by the addition of water.

B. A sample of 15% methanesulfonic acid (MSA) made according to Example 1A was electrolysed using a pure tin anode, an insoluble iridium oxide-coated titanium cathode. During electrolysis, tin dissolved into the 15% MSA. Trichloromethylmethylsulfone (TCMS) was added incrementally and the headspace gas above the electrochemical cell was smelled for detectable odor. After about 0.9 ppm of TCMS was added, an odor was noticeable.

### Example 2

A sample of 15% methanesulfonic acid (MSA) made as in Example 1(A) was electrolysed using a pure tin anode, an insoluble iridium oxide-coated titanium cathode.

During electrolysis, tin dissolved into the 15% MSA. Methylmethanethiosulfonate (MMTS) was incrementally added and the headspace gas above the electrochemical cell was smelled for detectable odor. After about 0.25 ppm of MMTS was added, an odor was noticeable. MMTS was seen to decompose using chromatographic techniques to primarily dimethyldisulfide (DMDS) in the electrolysis cell. Methylmercaptan (CH₃SH) and dimethylsulfide (DMS) were also observed after electrolysis, both odorous impurities.

### Example 3

A sample of 15% methanesulfonic acid (MSA) made as in Example 1(A) was electrolysed using a pure tin anode, an insoluble iridium oxide-coated titanium cathode.

During electrolysis, tin dissolved into the 15% MSA. Methylmethanethiosulfonate (MMTS) was incrementally added up to 0.85 ppm. An odor was detectable. To this solution was added 1.0 ppm of trichloromethylsulfone (TCMS). A very pungent odor was observed indicating a synergy between the two impurities in producing the undesirable odor.

### Example 4

A sample of 15% methanesulfonic acid (MSA) made as in Example 1(A) was electrolysed using a pure tin anode, an insoluble iridium oxide-coated titanium cathode.

During electrolysis, tin dissolved into the 15% MSA. Tricloromethylsulfone (TCMS) was added and after electrolysis, the MSA in the electrolysis cell contained dimethylsulfide and dimethyldisulfide leading to an undesirable odor.

### Example 5

A sample of 15% methanesulfonic acid (MSA) made as in Example 1(A) was electrolysed using a pure tin anode, an insoluble iridium oxide-coated titanium cathode.

During electrolysis, tin dissolved into the 15% MSA. After electrolysis, the MSA contained no undersirable low-valent sulfur compounds. A similar study was done but 1 ppm of MMTS was added to the MSA and after electrolysis, an odor was detected and the analysis showed dimethylsulfide, methylsulfide, dimethyldisulfide and dimethyltrisulfide were present in the MSA.

### Example 6

Two purities of methanesulfonic acid (MSA) were tested.

One was commercially available 70%MSA; the other 70%MSA of the present invention which was made according to the procedure of Example 1 except that it was not diluted.

The impurities, with amounts, in each composition is as follows:

| Component ID | Weight %, ppm (unless indicated) | |
|---|---|---|
| | 70% MSA of the invention | Commercial 70% MSA |
| DMDS | 0.05 | < 0.5 |
| MMS | None | |
| DMSO₂ | <5 | 20 |
| MCMS | 1 | 2 |
| MMTS | <1 | 10 |
| DCMS | 0.1 | < 0.5 |
| TCMS | None | 2 |
| Other Unknowns (total) | <1 | 4 |

A zinc methanesulfonate solution was prepared by dissolving zinc carbonate in 70% methanesulfonic acid as listed above. The final concentration of zinc ion was 65 g/l. During dissolution of zinc in the impure 70% MSA, a pungent odor was observed while the zinc solution prepared using the clean 70% MSA emitted no significant odor. An additional 1 molar free MSA was added to each zinc methanesulfonate solution, the same 70% MSA that was used to prepare the zinc electrolyte. Electrodeposition of zinc from each solution was done on clean steel panels at 65mA/cm², 60 amperes per square foot (ASF). The zinc deposit plated from the clean 70% MSAS electrolyte was matte gray and uniform while the zinc deposit plated from the impure 70% zinc solution was dark, rough and commercially unacceptable.

## Claims

1. Use in an electrochemical process of an aqueous solution comprising a methanesulfonic acid and low concentrations of low-valent sulfur (II) compounds and higher-valent sulfur (VI) compounds that are susceptible to reduction, wherein the total concentration of reduced sulfur compounds is less than 5 mg/liter, and wherein said low-valent sulfur (II) compounds and higher-valent sulfur (VI) compounds are chosen from methylmethanethiosulfonate (MMTS) and trichloromethylsulfone (TCMS).

2. The use of Claim 1, wherein the electrochemical process is chosen from electrodeposition, batteries, conductive polymers and descaling processes.

3. The use of Claim 1 wherein the concentration of the methanesulfonic acid is a ranges from 1 to 1480 g/l.

4. The use of Claim 3, wherein the concentration of the methanesulfonic acid is about 10 to about 700 grams per liter of solution.

5. The use of Claim 3, wherein the concentration of the methanesulfonic acid is about 30 to about 500 grams per liter of solution.

6. The use of Claim 1 wherein the pH is between 2 to 13.

7. The use of Claim 1 further comprising inorganic or organic acids in combination with methanesulfonic acid.

8. The use of Claim 1 further comprising a metal sulfonate salt or other metal salts and other free sulfonic acids.

9. The use of Claim 8 wherein the other metal salts is a salt of an alkyl or aryl sulfonic acid of formula: 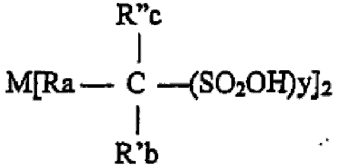 wherein a+b+c+y equals 4, M is a metal selected from metals in Group 1B, 2B, 3A, 3B, 4A, 4B, 5A, 5B, 6B, 7B, 8B, lanthanide metals of the periodic table, actinide metals of the periodic table, ammonium ion or mixtures thereof, R, R' and R" are the same or different and each independently is hydrogen, phenyl, Cl, F, Br, I, CF₃ or a C₁₋₂₃ alkyl group that is unsubstituted or substituted by oxygen, Cl, F, Br, I, CF₃, or -SO₂OH.

10. The use of Claim 8 wherein the other metal salts and free sulfonic acids are salts of or an alkyl sulfonic acid of formula: 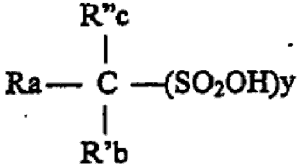 where in the other metal salts form via metal substitution on the -(SO₂OH)y group and_wherein a+b+c+y equals 4, R, R' and R" are the same or different and each independently are hydrogen, phenyl, Cl, F, Br, I, CF₃ or a C₁₋₂₃ alkyl group that is unsubstituted or substituted by oxygen, Cl, F, Br, I, CF₃, or - SO₂OH.

11. The use of Claim 8 wherein the metal sulfonate salt or other metal salts will be employed at a concentration from about 1 to about 600 grams per liter of aqueous solution.

12. The use of Claim 8 wherein the metal sulfonate salt or other metal salts is selected from metals in Group 1B, 2B, 3A, 3B, 4A, 4B, 5A, 5B, 6B, 7B, 8B, lanthanide or actinide metals of the periodic table and ammonium ion or mixtures thereof.

13. The use of Claim 8 wherein the metal sulfonate salt or other metal salts is zinc methanesulfonate salt.

14. The use of Claim 8 wherein the metal sulfonate salt or other metal salts is copper methanesulfonate salt.

15. The use of Claim 8 wherein the metal sulfonate salt or other metal salts is nickel methanesulfonate salt.

16. The use of Claim 8 wherein the metal sulfonate salt or other metal salts is ferrous methanesulfonate salt.

17. The use of Claim 8 wherein the metal sulfonate salt or other metal salts is an alkali or alkaline earth metal salt.

18. The use of Claim 8 wherein the metal sulfonate salt or other metal salts is a mixture of metal sulfonate salts selected from metals in Group 1B, 2B, 3A, 3B, 4A, 4B, 5A, 5B, 6B, 7B, 8B, lanthanide and actinide metals of the periodic table and ammonium ion.

19. The use of Claim 1 further comprising a buffering agent to modulate the pH of the aqueous solution.

20. The use of Claim 19 wherein the buffering agent is boric acid.

21. The use of Claim 1 further comprising an organic monomer selected from aniline or substituted aniline or pyrrole.

## Patentansprüche

1. Verwendung einer wässrigen Lösung, die eine Methansulfonsäure und geringe Konzentrationen von niederwertigen Schwefel(II)-Verbindungen und höherwertigen Schwefel(VI)-Verbindungen, die reduzierbar sind, bei einem elektrochemischen Prozess, wobei die Gesamtkonzentration von reduzierten Schwefelverbindungen weniger als 5 mg/Liter beträgt und wobei die niederwertigen Schwefel(II)-Verbindungen und höherwertigen Schwefel(VI)-Verbindungen aus Methylmethanthiosulfonat (MMTS) und Trichlormethylsulfon (TCMS) ausgewählt sind.

2. Verwendung nach Anspruch 1, wobei der elektrochemische Prozess aus elektrolytischer Abscheidung, Batterien, leitfähigen Polymeren und Entzunderungsverfahren ausgewählt ist.

3. Verwendung nach Anspruch 1, wobei die Konzentration der Methansulfonsäure im Bereich von 1 bis 1480 g/l liegt.

4. Verwendung nach Anspruch 3, wobei die Konzentration der Methansulfonsäure etwa 10 bis etwa 700 g/Liter Lösung beträgt.

5. Verwendung nach Anspruch 3, wobei die Konzentration der Methansulfonsäure etwa 30 bis etwa 500 g/Liter Lösung beträgt.

6. Verwendung nach Anspruch 1, wobei der pH zwischen 2 bis 13 liegt.

7. Verwendung nach Anspruch 1, ferner umfassend anorganische oder organische Säuren in Kombination mit Methansulfonsäure.

8. Verwendung nach Anspruch 1, ferner umfassend ein Metallsulfonatsalz oder andere Metallsalze und andere freie Sulfonsäuren.

9. Verwendung nach Anspruch 8, wobei sich bei den anderen Metallsalzen um ein Salz einer Alkyl- oder Arylsulfonsäure der Formel: 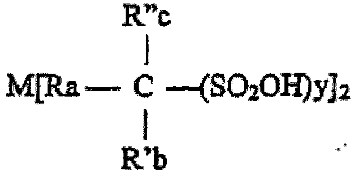 handelt, wobei a + b + c + y gleich 4 ist, M für ein Metall, das aus Metallen in Gruppe 1B, 2B, 3A, 3B, 4A, 4B, 5A, 5B, 6B, 7B, 8B, Lanthanidmetallen des Periodensystems, Actinidmetallen des Periodensystems ausgewählt ist, ein Ammoniumion oder Mischungen davon steht, R, R' und R'' gleich oder verschieden sind und jeweils unabhängig für Wasserstoff, Phenyl, Cl, F, Br, I, CF₃ oder eine C₁₋₂₃-Alkylgruppe, die unsubstituiert oder durch Sauerstoff, Cl, F, Br, I, CF₃ oder -SO₂OH substituiert ist, stehen.

10. Verwendung nach Anspruch 8, wobei sich bei den anderen Metallsalzen und freien Sulfonsäuren um Salze einer Alkylsulfonsäure oder eine Alkylsulfonsäure der Formel: 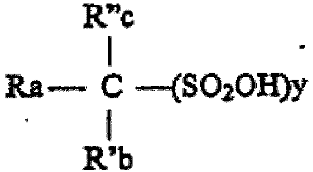 handelt, wobei die anderen Metallsalzen durch Metallsubstitution an der -(SO₂OH)_{y}-Gruppe gebildet werden und wobei a + b + c + y gleich 4 ist, R, R' und R'' gleich oder verschieden sind und jeweils unabhängig für Wasserstoff, Phenyl, Cl, F, Br, I, CF₃ oder eine C₁₋₂₃-Alkylgruppe, die unsubstituiert oder durch Sauerstoff, Cl, F, Br, I, CF₃ oder -SO₂OH substituiert ist, stehen.

11. Verwendung nach Anspruch 8, wobei das Metallsulfonatsalz oder die anderen Metallsalze in einer Konzentration von etwa 1 bis etwa 600 Gramm pro Liter wässrige Lösung eingesetzt werden.

12. Verwendung nach Anspruch 8, wobei das Metallsulfonatsalz oder die anderen Metalle aus Metallen in Gruppe 1B, 2B, 3A, 3B, 4A, 4B, 5A, 5B, 6B, 7B, 8B, Lanthanid- oder Actinidmetallen des Periodensystems und Ammoniumionen oder Mischungen davon ausgewählt sind.

13. Verwendung nach Anspruch 8, wobei es sich bei dem Metallsulfonatsalz oder den anderen Metallsalzen um Zinkmethansulfonatsalz handelt.

14. Verwendung nach Anspruch 8, wobei es sich bei dem Metallsulfonatsalz oder den anderen Metallsalzen um Kupfermethansulfonatsalz handelt.

15. Verwendung nach Anspruch 8, wobei es sich bei dem Metallsulfonatsalz oder den anderen Metallsalzen um Nickelmethansulfonatsalz handelt.

16. Verwendung nach Anspruch 8, wobei es sich bei dem Metallsulfonatsalz oder den anderen Metallsalzen um Eisen(II)-methansulfonatsalz handelt.

17. Verwendung nach Anspruch 8, wobei es sich bei dem Metallsulfonatsalz oder den anderen Metallsalzen um ein Alkali- oder Erdalkalimetallsalz handelt.

18. Verwendung nach Anspruch 8, wobei es sich bei dem Metallsulfonatsalz oder den anderen Metallsalzen um eine Mischung von Metallsulfonatsalzen, die aus Metallen in Gruppe 1B, 2B, 3A, 3B, 4A, 4B, 5A, 5B, 6B, 7B, 8B, Lanthanid- oder Actinidmetallen des Periodensystems und Ammoniumionen ausgewählt sind, handelt.

19. Verwendung nach Anspruch 1, ferner umfassend eine Puffersubstanz zur Modulierung des pH-Werts der wässrigen Lösung.

20. Verwendung nach Anspruch 19, wobei sich bei der Puffersubstanz um Borsäure handelt.

21. Verbindung nach Anspruch 1, ferner umfassend ein organisches Monomer, das aus Anilin oder substituiertem Anilin oder Pyrrol ausgewählt ist.

## Revendications

1. Utilisation dans un procédé électrochimique d'une solution aqueuse comprenant un acide méthanesulfonique et de faibles concentrations de composés de soufre(II) de faible valence et de composés de soufre(VI) de valence plus élevée qui sont susceptibles de subir une réduction, dans laquelle la concentration totale de composés du soufre réduits est inférieure à 5 mg/litre et dans laquelle lesdits composés de soufre (II) de faible valence et composés de soufre(VI) de valence plus élevée sont choisis entre le méthanethiosulfonate de méthyle (MMTS) et la trichlorométhylsulfone (TCMS).

2. Utilisation selon la revendication 1, dans laquelle le procédé électrochimique est choisi parmi un dépôt électrolytique, des batteries, des polymères conducteurs et des procédés de décalaminage.

3. Utilisation selon la revendication 1 dans laquelle la concentration de l'acide méthanesulfonique est dans une plage de 1 à 1480 g/l.

4. Utilisation selon la revendication 3, dans laquelle la concentration de l'acide méthanesulfonique est d'environ 10 à environ 700 grammes par litre de solution.

5. Utilisation selon la revendication 3, dans laquelle la concentration de l'acide méthanesulfonique est d'environ 30 à environ 500 grammes par litre de solution.

6. Utilisation selon la revendication 1 dans laquelle le pH est compris entre 2 et 13.

7. Utilisation selon la revendication 1 comprenant en outre des acides inorganiques ou organiques en association avec de l'acide méthanesulfonique.

8. Utilisation selon la revendication 1 comprenant en outre un sel sulfonate métallique ou d'autres sels métalliques et d'autres acides sulfoniques libres.

9. Utilisation selon la revendication 8 dans laquelle les autres sels métalliques sont un sel d'un acide alkylsulfonique ou arylsulfonique de formule : 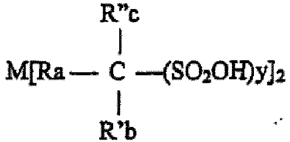 dans laquelle a + b + c + y est égal à 4, M est un métal choisi parmi les métaux du groupe 1B, 2B, 3A, 3B, 4A, 4B, 5A, 5B, 6B, 7B, 8B, les métaux des lanthanides du tableau périodique, les métaux des actinides du tableau périodique, l'ion ammonium ou les mélanges de ceux-ci, R, R' et R" sont identiques ou différents et sont chacun indépendamment l'atome d'hydrogène, un groupe phényle, Cl, F, Br, I, CF₃ ou un groupe alkyle en C₁₋₂₃ qui est non substitué ou substitué par l'atome d'oxygène, Cl, F, Br, I, CF₃ ou -SO₂OH.

10. Utilisation selon la revendication 8 dans laquelle les autres sels métalliques et acides sulfoniques libres sont un acide alkylsulfonique ou des sels de celui-ci de formule : 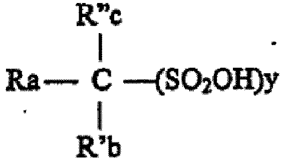 dans laquelle les autres sels métalliques se forment par substitution métallique sur le groupe -(SO₂OH)_{y} et dans laquelle a + b + c + y est égal à 4, R, R' et R" sont identiques ou différents et sont chacun indépendamment l'atome d'hydrogène, un groupe phényle, Cl, F, Br, I, CF₃ ou un groupe alkyle en C₁₋₂₃ qui est non substitué ou substitué par l'atome d'oxygène, Cl, F, Br, I, CF₃ ou -SO₂OH.

11. Utilisation selon la revendication 8 dans laquelle le sel sulfonate métallique ou les autres sels métalliques seront employés en une concentration d'environ 1 à environ 600 grammes par litre de solution aqueuse.

12. Utilisation selon la revendication 8 dans laquelle le sel sulfonate métallique ou les autres sels métalliques sont choisis parmi les métaux du groupe 1B, 2B, 3A, 3B, 4A, 4B, 5A, 5B, 6B, 7B, 8B, les métaux des lanthanides ou des actinides du tableau périodique et l'ion ammonium ou les mélanges de ceux-ci.

13. Utilisation selon la revendication 8 dans laquelle le sel sulfonate métallique ou les autres sels métalliques sont le sel méthanesulfonate de zinc.

14. Utilisation selon la revendication 8 dans laquelle le sel sulfonate métallique ou les autres sels métalliques sont le sel méthanesulfonate de cuivre.

15. Utilisation selon la revendication 8 dans laquelle le sel sulfonate métallique ou les autres sels métalliques sont le sel méthanesulfonate de nickel.

16. Utilisation selon la revendication 8 dans laquelle le sel sulfonate métallique ou les autres sels métalliques sont le sel méthanesulfonate ferreux.

17. Utilisation selon la revendication 8 dans laquelle le sel sulfonate métallique ou les autres sels métalliques sont un sel de métal alcalin ou alcalinoterreux.

18. Utilisation selon la revendication 8 dans laquelle le sel sulfonate métallique ou les autres sels métalliques sont un mélange de sels sulfonates métalliques choisis parmi les métaux du groupe 1B, 2B, 3A, 3B, 4A, 4B, 5A, 5B, 6B, 7B, 8B, les métaux des lanthanides et des actinides du tableau périodique et l'ion ammonium.

19. Utilisation selon la revendication 1 comprenant en outre un agent tampon pour moduler le pH de la solution aqueuse.

20. Utilisation selon la revendication 19 dans laquelle l'agent tampon est l'acide borique.

21. Utilisation selon la revendication 1 comprenant en outre un monomère organique choisi entre l'aniline, l'aniline substituée et le pyrrole.
